Europäisches Patentamt

European Patent Office

Office européen des brevets

(11) Veröffentlichungsnummer: **0 161 471**
**B1**

(12) EUROPÄISCHE PATENTSCHRIFT

(45) Veröffentlichungstag der Patentschrift: **09.01.91**

(51) Int. Cl.⁵: **A 61 J 1/00**

(21) Anmeldenummer: **85104160.8**

(22) Anmeldetag: **04.04.85**

(54) Behälter für eine bicarbonathaltige Flüssigkeit.

(30) Priorität: **06.04.84 IT 8555484**

(43) Veröffentlichungstag der Anmeldung:
**21.11.85 Patentblatt 85/47**

(45) Bekanntmachung des Hinweises auf die
Patenterteilung:
**09.01.91 Patentblatt 91/02**

(84) Benannte Vertragsstaaten:
**AT BE CH DE FR GB LI NL SE**

(56) Entgegenhaltungen:
EP-A-0 022 922
EP-A-0 050 255
EP-A-0 083 778
WO-A-83/00430
CH-A- 364 073
DE-U-7 719 528

The International Journal of Artificial Organs,
1981, S.308 und 309 und 1983, Vol.6, Nr. 4,
Seiten 217 und 218

(73) Patentinhaber: **Fresenius AG**
**Gluckensteinweg 5**
**D-6380 Bad Homburg v.d.H. (DE)**

(72) Erfinder: **Feriani, Mariano, Dr.**
**Via Gregori 7/8**
**Arcugnano (Vicenza) (IT)**
Erfinder: **Biasioli, Stefano, Dr.**
**Via Marosticana 397**
**Vicenza (IT)**

(74) Vertreter: **Luderschmidt, Wolfgang, Dr. Dipl.-**
**Chem.**
**Dr. Fuchs, Dr. Luderschmidt, Dipl.-Phys. Seids,**
**Dr. Mehler Patentanwälte Abraham-Lincoln-**
**Strasse 7 Postfach 46 60**
**D-6200 Wiesbaden (DE)**

Courier Press, Leamington Spa, England.

**Beschreibung**

Die Erfindung betrifft einen Behälter zur Bereitstellung einer bicarbonhaltigen Dialysier-, Substitutions- oder Infusionsflüssigkeit für die Peritonealdialyse, Hämofiltration bzw. Infusion, aufweisend einen ersten Behälterteil, der eine Säurelösung aufweist, und einen zweiten Behälterteil, der mit dem ersten Behälterteil über ein abgesperrtes, jedoch zu öffnendes Strömungsverbindungsteil verbunden ist und der mit einer bicarbonathaltigen Lösung gefüllt ist, wobei einer der Behälterteile wenigstens ein Auslaßrohr aufweist, das mit einem entfernbaren Verschluß versehen ist.

Bei der kontinuierlichen ambulanten Peritonealdialyse (CAPD) wird eine Dialysierflüssigkeit in den Peritonealraum des Patienten eingeführt, wobei diese Dialysierflüssigkeit eine für die Dialyse notwendige Elektrolytzusammensetzung aufweist und als Pufferelement Acetat- oder Lactationen zur Behandlung der metabolischen Acidose besitzt. Eine derartige Lösung ist jedoch ebenfalls schwach sauer und hemmt die im Peritoneum vorliegenden antimikrobiellen Körper, so daß die Einführung derartiger Lösungen in den Peritonealraum unter strikt sterilen Bedingungen durchgeführt werden muß, da ansonsten die Gefahr einer Peritonitis besteht.

Aufgrund dieser Erkenntnisse hat es bereits Versuche gegeben, bicarbonathaltige Lösungen für die Peritonealdialyse zur Verfügung zu stellen, um auch im Peritoneum möglichst physiologische Bedingungen zu schaffen, was u.a. zur Folge hat, daß die Hemmung der antimikrobiellen Körper aufgehoben wird.

Dabei ist der Einsatz einer bicarbonathaltigen Dialysierflüssigkeit beispielsweise aus der EP—A—22 922 bekannt, die dadurch hergestellt wird, daß man ein Säurekonzentrat mit einem Bicarbonatkonzentrat unter Hinzufügung von Wasser vermischt. Es müssen insofern zwei Konzentrate eingesetzt werden, als die Gefahr besteht, daß durch die Reaktion von Calciumionen mit Carbonationen unlösliches Calciumcarbonat ausgefällt wird, das für den physiologischen Bedarf nicht mehr zur Verfügung steht.

Diese Lösungen sind jedoch für die Einführung in das Peritoneum nicht ausreichend steril, so daß nach weiteren Lösungsmöglichkeiten dieses Problems gesucht worden ist.

So haben T. S. Ing et al in Int. J. Artif. Organs 1981, S.308, 309 und 1983, S.217, 218 die On-line-Herstellung einer bicarbonathaltigen Dialysierflüssigkeit zur Verwendung in der Peritonealdialyse vorgeschlagen, wobei eine saure Lösung und eine basische Lösung vermischt und das hieraus erhaltene Dialysatprodukt anschließend dem Patienten zugeführt wird. Dabei wird die basische Lösung, die das Njb catriumbicarbonat enthält, grundsätzlich in einem Glasbehälter, beispielsweise einer Glasfasche oder einer Glasspritze, vorgelegt, in dem grundsätzlich nicht die Gefahr besteht, daß evtl. aus dem Bicarbonat während der Lagerzeit abgespaltenes $CO_2$ durch Diffusion verschwindet. Infolgedessen bleibt also in dem Glasgefäß die ursprünglich eingesetzte bicarbonathaltige Lösung vollständig erhalten und kann somit vollständig bei der Herstellung einer bicarbonathaltigen Dialysierflüssigkeit eingesetzt werden.

Medizinische Lösungen müssen in aller Regel mindestens etwa ein Jahr lagerfähig und stabil sein, d.h. während dieses Zeitraums darf sich die Zusammensetzung einer derartigen Lösung nicht verändern. Das Bicarbonat-Ion liegt jedoch im Gleichgewicht mit dem $OH^-$-Ion und $CO_2$, das zwar physikalisch in der wässrigen Lösungen gelöst werden kann, jedoch aber auch durch Diffusion aus dem Behälter entfernt werden kann, sofern hierzu der Behälter eine Diffusionsmöglichkeit bietet. Zusätzlich baut sich durch die Zersetzung des Bicarbonats und die Freisetzung von $CO_2$ im Behälter ein Innendruck auf, der eine gewisse Stabilität des Behälters erfordet, da dieser ansonsten platzt.

Insofern haben also Ing et al einen Glasbehälter zum Einsatz der bicarbonathaltigen Lösung vorgeschlagen, wie er üblicherweise zur Aufnahme von mit $CO_2$ gesättigten Mineralwässern zum Einsatz kommt.

Der Einsatz einer derartigen Anordnung ist jedoch nicht für die CAPD geeignet, da hier üblicherweise ein Beutel am Körper des Patienten getragen wird, der zur Abgabe der frischen Dialysierflüssigkeit über ein Schlauchsystem in den Peritonealraum des Patienten und zur Aufnahme der verbrauchten Dialysierflüssigkeit dient.

Der Erfindung liegt daher die Aufgabe zugrunde, den Behälter der eingangs erwähnten Art so fortzubilden, daß er problemlos bei der CAPD, Hämofiltration oder Infusion eingesetzt werden kann, ohne daß die Gefahr besteht, daß sich die Zusammensetzung der bicarbonathaltigen Lösung praktisch verändert.

Die Lösung der Aufgabe erfolgt dadurch, daß der erste Behälterteil und der zweite Behälterteil in einer wenigstens zwei Kammern aufweisenden Beutelanordnung aus einem organischen Polymerisat vorgesehen sind.

Überraschenderweise läßt sich der erfindungsgemäße Behälter zur Langzeitspeicherung von bicarbonathaltigen Lösungen einsetzen, d.h. es besteht praktisch nicht die Gefahr, daß das durch die Zersetzung von Bicarbonat freigewordene $CO_2$ im nennenswerten Umfang durch die polymere Wand des Beutels hindurchdiffundiert. Dies ist insofern überraschend, als man bisher grundsätzlich davon ausging, daß entweder dickwandige Kunststoffbehälter oder aber gasdichte Glasbehälter eingesetzt werden müssen, um eine wirksame Diffusionssperre gegenüber dem freigesetzten $CO_2$ zu bilden.

Mit dem erfindungsgemäßen Behälter kann nun erstmals ein Beutel für eine bicarbonathaltige Lösung eingesetzt werden, der eine ausreichende Langzeitstabilität aufweist, also wenigstens etwa in Jahr die ursprünglich eingesetzte Bicarbonatlösung ohne Zersetzung speichern kann.

Des weiteren läßt sich die erfindungsgemäße Beutelanordnung besonders vorteilhaft bei der CAPD einsetzen, da die bicarbonathaltige Dialysierflüssigkeit unmittelbar vor der Einführung in den Peritonealraum hergestellt werden kann und anschließend die gesamte Beutelanordnung bequem am Körper des Patienten getragen werden kann. Hieraus ergeben sich sowohl für den Benutzer als auch für die Durchführung der CAPD selbst Vorteile, da einerseits der Patient selbst von einer Dialysemaschine unabhängig ist und andererseits die erhaltene bicarbonathaltige Dialysierflüssigkeit den erwünschten physiologischen pH-Wert aufweist, der die natürlichen Abwehrkräfte des Peritoneums nicht hemmt.

Festzustellen ist also, daß die Ausfällung von Calciumcarbonat durch den Einsatz des erfindungsgemäßen Beutels verhindert wird und daß die bereitgestellten Lösungen ohne Schwierigkeiten herzustellen und zu sterilisieren sind und weiterhin über den notwendigen Zeitraum in dem eingesetzten Kunststoffmaterial gelagert werden können.

In dem erfindungsgemäßen Behälter sind wenigstens zwei Behälterteile oder Compartments vorgesehen, die miteinander über ein Strömungsverbindungsteil verbunden sind. Dieses Strömungsverbindungsteil ist üblicherweise rohrförmig ausgebildet und kann entweder starrer oder flexibler Natur sein.

Bei starrer Ausbildung des Strömungsverbindungsteils wird üblicherweise ein Kunststoffrohr aus einem starren Material gewählt, das jeweils an seinen Enden mit den beiden Behälterteilen verbundun ist. Andererseits kann jedoch aber auch ein derartiges Verbindungsteil vollständig von dem Kunststoffmaterial der beiden Compartimente umgeben sein, was besonders vorteilhaft ist.

In ähnlicher Weise kann jedoch aber auch ein flexibles Rohrstück, beispielsweise in Form eines Schlauchstücks, eingesetzt werden, das mit den jeweiligen Behälterteilen, beispielsweise durch Verschweißen, verbunden ist. Ein derartiges flexibles Schlauchstück kann wie das starre Strömungsverbindungsteil entweder ganz oder teilweise mit dem jeweiligen Kunststoffmaterial der beiden Behälterteile verbunden sein.

Das vorstehend erwähnte Strömungsverbindungsteil ist vor dem Vermischen der beiden in den Behälterteilen befindlichen Lösungen verschlossen. Insofern ist erfindungsgemäß das Strömungsverbindungsteil mit einem Absperrorgan versehen, das im Gebrauchsfall geöffnet bzw. entfernt wird.

Als derartiges Absperrorgan kommen beispielsweise die üblicherweise eingesetzten Abbrechteile bei der starren Ausführungsform in Frage, die ein rohrförmiges Teil verschließen und bei Gebrauch entlang einer Schwächungslinie von diesem rohrförmigen Teil abgebrochen werden können. Insofern handelt es sich hier um ein integral mit den beiden Behälterteilen verbundenes Absperrorgan, das vorteilhafterweise an dem ebenfalls integral mit den beiden Behälterteilen verbundenen Strömungsverbindungsteil vorgesehen ist.

Weiterhin kann natürlich auch das Strömungsverbindungsteil mit einer Sperrwand versehen sein, die im Bedarfsfall zerstört wird, so daß hierdurch die Strömungsverbindung hergestellt wird. So kann beispielsweise ein Rohr aus einem starren Kunststoffmaterial wiederum zwischen den beiden Behälterteilen vorgesehen sein, dessen Durchströmöffnung mit einer derartigen Wand aus dem gleichen Kunststoffmaterial verschlossen ist. Diese Sperrwand wird im Einsatzfall von einer entsprechend ausgebildeten Öffnungseinrichtung, beispielsweise einem Dorn (Spike) durchstoßen, wodurch die Strömungsverbindung zwischen den beiden Beutelteilten hergestellt wird.

Schließlich kann aber auch das Strömungsverbindungsteil durch eine Verschlußeinrichtung verschlossen sein, die mittels Reibschluß, beispielsweise in Form eines elastischen Stopfens, das Strömungsverbindungsteil absperrt. Ein solcher Verschluß ist insbesondere in dem Behälterteil einzusetzen, in dem ein gewisser Überdruck herrschen kann. Erfindungsgemäß wird man also den Stopfen von der Seite des Behälterteils her, her die Bicarbonatlösung aufweist, in den Strömungsverbindungsteil einsetzen.

Anstelle eines Verbindungsstücks aus einem starren Kunststoff, beispielsweise Polycarbonat u.dgl., kann jedoch aber auch ein Verbindungsstück aus einem weichen Kunststoffmaterial, beispielsweise Weich-PVC oder Polyethylen u.dgl. eingesetzt werden, das entweder wiederum mit einem durch Reibschluß verbundenen Absperrteil verschlossen ist oder von außen her mit einer Klemmeinrichtung abgeklemmt ist. Des weiterhin kann in dem flexiblen Strömungsverbindungsteil auch ein starres Kunststoffabsperrteil integral oder durch Reibschluß vorgesehen sein, das — wie vorstehend erwähnt — ein Abbrechteil aufweist, das zum Vermischen der beiden Lösungen abgebrochen wird.

Besonders bevorzugt ist das integrale Einschmelzen eines starren Rohrstücks, dessen Öffnung mit einem Abbrechteil entlang einer Schwächungslinie ebenfalls integral verbunden ist. Dieses nachstehend als Abbrechteil beschriebene Verschlußteil ist vorteilhafterweise in einer Zwei-Kammer-Beutelanordnung vorgesehen, bei der die beiden Kammern in einem einstückig ausgebildeten Beutel vorgesehen und durch eine Schweißnaht voneinander getrennt sind. In diese Schweißnaht ist das Abbrechteil eingefügt und schafft somit eine Strömungsverbindung zwischen den beiden Kammern nach dem Zerstören das Abbrechteils.

Ein derartiger Zwei-Kammer-Beutel wird dadurch hergestellt, daß man zwei Kunststoff-Folien entlang eines Außenrandes miteinander unter Freilassung von Füllschlitzen bzw. Schlitzen zur Aufnahme eines Ablaßschlauchs verschweißt und zusätzlich quer über den Beutel eine Schweißnaht unter Teilung des Beutels in eine erste und eine zweite Kammer anbringt, wobei in diese querverlaufende Schweißnaht ein Strömungsverbindungsteil eingefügt ist. Durch die Füllschlitze, in die gegebenenfalls Füllstutzen eingeschweißt sein können, werden die zu lagernden Lösungen in die Kammern eingefüllt. Anschließend werden die Füllschlitze bzw. Füllstutzen abgeschweißt d.h. die Kammern werden gegenüber der Atmosphäre abgeschlossen. Zuvor ist sichergestellt, daß der Ablaßschlauch ebenfalls verschlossen ist.

Als Beutelmaterial werden organische Polymere eingesetzt, die über eine geringe Wasserdampfdurchlässigkeit bzw. Kohlendioxiddurchlässigkeit verfügen. Zu einsetzbaren Polymeren gehören Polyethylen, Polypropylen, PVC, Polyvinylidenchlorid, Polymethylmetacrylat sowie Copolymerisate und Mischpolymerisate, beispielsweise Ethylen/Propylen-Kunststoffe, Poly-(Ethylen/Vinylacetat), Acrylnitril/Butadien/Styrol-Polymerisate, Ethylen-Propylen-Block-Copolymerisat, Styrolcopolymerisate u.dgl.

Sofern PVC eingesetzt wird, soll diese vorteilhafterweise nur Weichmacher auf organischer Basis, beispielsweise Dioctylphthalat, enthalten.

Bevorzugt Beutelmaterialien, die mit den Lösungen in Berührung kommen, sind Polyethylen und PVC.

Zur Verringerung der Wasserdampfdurchlässigkeit und der Kohlendioxiddurchlässigkeit der vorstehend genannten Polymerisate kann das als Beutelfolie eingesetzte Polymerisat auf seiner Außenseite mit einer oder mehreren diese Durchlässigkeiten senkenden Schicht(en) in Form eines Laminats beschichtet sein. Als derartige Laminatschicht auf der Beutelfolie kann beispielsweise eine Metallfolie oder eine weiteres Polymerisat eingesetzt werden, beispielsweise Polyamide, PVC, Polyvinylidenchlorid, Polyvinylfluorid, Polytrifluorchlorethylen, Polyethylenterphthalat, Polyester u.dgl. Bevorzugt sind Polyamid, Polyvinylidenchlorid, Polyethylenterphthalat und Polyester.

Die polymeren Außen- und Innenfolien werden vorteilhafterweise mittels eines Kaschierklebers, wie Polyvinylidenchlorid oder Polyurethan, zusammengeklebt und liegen danach als einsatzfähige Laminate vor.

Bevorzugte Laminate weisen bei Raumtemperatur und einer relativen Luftfeuchtigkeit von etwa 85% in der Regel eine Wasserdampfdurchlässigkeit nach DIN 53 122 von <1 auf.

Derartige Werte gelten für Standardfolien mit einer Stärke von 50—100 μm, insbesondere etwa 75 μm für die Innenfolie und 20—100 μm, insbesondere 30—70 μm für die als Außenfolie dienende Kaschierfolie.

Weiterhin ist bei den bevorzugten Laminaten die Kohlendioxiddurchlässigkeit gesenkt, wobei dieser Wert unterhalb 20 $cm^3$/,$^2$ × Tag × bar Druckdifferenz liegt.

Als besonders vorteilhaft läßt sich ein Laminat sowie ein hieraus hergestellter Zwei-Kammer-Beutel einsetzten, wie er prinzipiell in der DE—A—32 00 263 beschrieben ist, auf deren Offenbarung Bezug genommen wird.

Hiernach wird als Innerfolie ein Polyethylen mit mittlerer bis hoher Dichte eingesetzt, die üblicherweise durch Niederdruckpolymerisation hergestellt wird. Dabei liegt die Dichte in einem Bereich von 0,91—0,94, insbesondere bei etwa 0,935 g/$cm^3$. Dieses Polyethylen läßt sich ohne Schwierigkeiten bei der Sterilisationstemperatur von etwa 115—125°C einsetzen.

Weiterhin ist dieses Polyethylen vorteilhafterweise mit einer Polyamidfolie in den vorstehend erwähnten Stärken kaschiert. Derartige Folien werden beispielsweise unter der Bezeichnung "Flexovac V 7144" von der Firma Sengewald, Halle/Westf., BR Deutschland, für medizinische Zwecke vertrieben.

Vorteilhafterweise ist in diese Polyethylen-Innenfolie ein Auslaßschlauch aus einem Copolymerisat von Ethylen und Vinylacetat (EVA) eingeschweißt, der anschließend durch hochenergetische Strahlung kreuzvernetzt wird und hierdurch unter Aufrechterhaltung seiner elastischen Eigenschaften bei den vorstehend genannten Sterilisationstemperaturen ohne Zerstörung eingesetzt werden kann.

Hinzufügen ist, daß auch das Strömungsverbindungsteil aus kreuzvernetztem EVA bestehen kann, sofern ein flesibles Polymerisat verwendet werden soll.

Gemäß einer weiterhen Ausführungsform kann PVC verwendet werden, das das übliche Beutelmaterial für medizinische Zwecke darstellt. In einen derartigen Beutel können PVC-Schlauchstücke sowohl als Strömungsverbindungsteil als auch als Auslaßschlauch eingeschweißt sein.

Diese PVC-Innenfolie kann — wie bereits vorstehenden erwähnt — zur Verminderung oder Durchlässigkeiten von Wasserdampf und $CO_2$ mit einer Außenfolie kaschiert sein, deren Material vorstehend genannt worden ist.

In einer weiteren, bevorzugten Ausführungsform, die insbesondere bei Beutelmaterialien eingesetzt wird, die eine erhöhte $CO_2$-Durchlässigkeit aufweisen, wird die gesamte Beutelanordnung einschließlich der Ablaßleitung und des an dieser Ablaßleitung befindlichen Konnektorstücks mit einer Schutzhülle aus einem Material umgeben, das vorteilhafterweise eine sehr geringe $CO_2$-Durchlässigkeit aufweist. Diese Schuthülle ist vorteilhafterweise aus einem weichen, durchsichtigen Kunststoff, der für Keime undurchdringlich ist und darüber hinaus bei wenigstens 115—125°C sterilisierbar ist. Zwischen dieser Schutzhülle, die die Außenbahn des vorstehend genannten Laminats ersetzt, und der erfindungsgemäßen Beutelanordnung kann vorteilhafterweise gasförmiges $CO_2$ in einem solchen Druck vorgesehen sein, daß der $CO_2$-Partialdruck in der bicarbonathaltigen Flüssigkeit zumindest kompensiert wird. Hierdurch wird also eine Zersetzung von Bicarbonat gehemmt. Eine solche Anordnung ist insbesondere in den Fällen bevorzugt, bei denen ein üblicher PVC-Beutel, der eine Wandstärke von etwa 0,4—0,6 mm besitzt, ohne Kaschierung mit einer Außenfolie zur Aufnahme der beiden Flüssigkeiten dient.

Derartige Schutzhüllen werden vorzugsweise in Form von Laminaten eingesetzt, beispielsweise Laminate aus Polyester und Polypropylen, Polyamid und Polypropylen oder Polyethylenterephthalat und Polypropylen.

Eine derartige Schutzhülle besteht aus einer Oberbahn und Unterbahn, die unter Bildung eines Beutel an den Rändern verschweißt werden. Gegebenenfalls kann eine de Bahnen oder beide Bahnen entsprechend der Form des zu schütztenden Beutels tiefgezogen sein, wie dies in der EP—A—50 255 beschrieben ist, auf deren Offenbarung ausdrücklich Bezug genommen wird.

Weiterhin kann eine derartige Schutzhülle, sofern diese aus einem Material besteht, das eine sehr geringe $CO_2$-Durchlässigkeit aufweist, auch entsprechend dem Verfahren der Europäischen Patentschrifft evakuiert sein und somit eng an dem du schützenden Beutel anliegen. Demzufolge liegt also zwischen den beiden Beuteln praktisch kein gasförmiges $VO_2$ vor.

Schließlich kann auch anstelle von Laminaten aus Kunststoffmaterialien auch ein Mischmaterial aus einer Kunststoffschicht und einer Metallfolie, beispielsweise einer Aluminiumfolie, eingesetzt werden, die praktisch in den eingesetzten Stärken $CO_2$-undurchlässig ist, so daß lediglich an den Kunststoffverschweißungsstellen $CO_2$ hindurch-diffundieren kann, was jedoch im wesentlichen vernachlässigbar ist.

Die Füllvolumina der beiden Behälterteile werden entsprechend dem Einsatzzweck gewählt, wobei das Volumen eines jeden Behälterteils vorteilhafterweise von 0,5—2,5 Liter variieren kann. Dabei besitzt vorteilhafterweise das die endgültige Mischung aufnehmende Compartment eine solche Größe, daß beide Flüssigkeiten zusammen aufgenommen werden können. Andererseits lassen sich jedoch aber auch beide Flüssigkeiten durch wechselweise erfolgendes Hin- und Her-Pumpen in der Beutelanordnung miteinander vermischen.

Die saure Lösung, die die Calcium- und Magnesiumsalze enthält, hat erfindungsgemäß folgende Konzentration, ausgedrückt in mval (mEq)/l Was-ser:

$$Ca^{2+} = 1—10$$

$$Mg^{2+} = 0—6$$

$$Cl^- = 1—16$$

$$CH_3COOH = 4—6$$

Die bicarbonathaltige Lösung weist folgende Bestandteile in mval/1 Wasser auf:

$$Na^+ = 256—290$$

$$K^+ = 0—8$$

$$HCO_3^- = 56—76$$

$$Cl^- = 180—238$$

Anstelle von Natriumhydrogencarbonat in der bicarbonathaltigen Lösung kann auch Natriumcarbonat eingesetzt werden, wobei die basische Lösung dann 120—128 mval/l Carbonat und die saure Lösung 60—64 mval/l HCl aufweisen.

Beide Lösungen werden miteinander im Verhältnis von 1:1 vermischt, wobei die endgültige Lösung folgende Zusammensetzung in mval/l aufweist:

$$Ca^{2+} = 0,5—5$$

$$Mg^{2+} = 0—3$$

$$Cl^- = 90,5—121$$

$$CH_3COOH = 2—3$$

$$Na^+ = 128—145$$

$$K^+ = 0—4$$

$$HCO_3^- = 28—38$$

Die in der Mischung befindliche Essigsäure reagiert mit dem Hydrogen-Carbonation unter Freisetzung von 2—3 mmol/l $CO_2$, das in dem Gemisch physikalisch gelöst wird, wobei ein bestimmter Überdruck in der Lösung entsteht Dieser ist abhängig vom Partialdruck $P_{CO_2}$ und liegt etwa zwischen 50—80 mm/Hg.

Sofern Natriumcarbonat als basisches Agens eingesetzt wird, reagiert dieses mit der Salzsäure der sauren Lösung unter Bildung von $CO_2$ und Hydrogencarbonationen in etwa gleichen Men-gen. Auch diese $CO_2$-Menge kann von dem Beutel aufgegangen werden.

Eine derartige Lösung kann infolge ihrer isotonischen Eigenschaften sowohl für die Dialyse und Hamöfiltration als auch für Infusionszwecke eingesetzt werden.

Falls die bicarbonathaltige Lösung auch osmotisch wirksam sein soll, was üblicherweise bei Lösungen für die CAPD der Fall ist, ist eine bestimmte Menge einer osmotisch wirksamen Substanz, beispielsweise Glucose, in der sauren Lösung vorgesehen. Im vorliegenden Fall enthält die saure Lösung etwa 26—90 g Glucose/l, was bei der 1:1-Verdünnung zu einer Osmolarität der Lösung von etwa 350—550 mosm/l führt.

Infolge der stark alkalischen Eigenschaften einer Natriumcarbonatlösung und der starken Entwicklung von $CO_2$ bei der Umsetzung mit der sauren Lösung ist der Einsatz von Natriumhydrogencarbonat gegenüber dem Einsatz von Natriumcarbonat (Soda) bevorzugt.

Als besonders vorteilhaft hat sich herausgestellt, daß die basische, bicarbonathaltige Lösung, die noch nicht mit der Säurelösung vermischt ist, in dem Compartment vorgelegt wird, das die Ablaßleitung oder -schlauch aufweist. Diese Anordnung ist aus Sicherheitsgründen zu bevorzugen, da bei der ambulanten, d.h. vom Patienten selbst durchgeführten Dialyse, u.U. die Gefahr besteht, daß eine noch nicht vermischte Lösung durch die Ablaßeitung in den Peritonealraum des Patienten gelangt. Eine bicarbonathaltige Lösung im unvermischten Zustand kann vom Patienten ohne Schwierigkeiten toleriert erden, während dies bei der Säurelösung nicht der Fall ist. Insofern ist also die Vorlage der Bicarbonatlösung in dem Compartment, das mit der Ablaßleitung verbunden ist, bevorzugt. Hinzuzufügen ist, daß der Stand der Technik (vgl. Ing. 1983) die Füllung eines Kunststoffbeutels mit der Säurelösung vorschlägt, was aus Sicherheitsgründen nachteilig ist.

Die Erfindung wird anhand eines Ausführungsbeispiels erläutert.

Es zeigen:

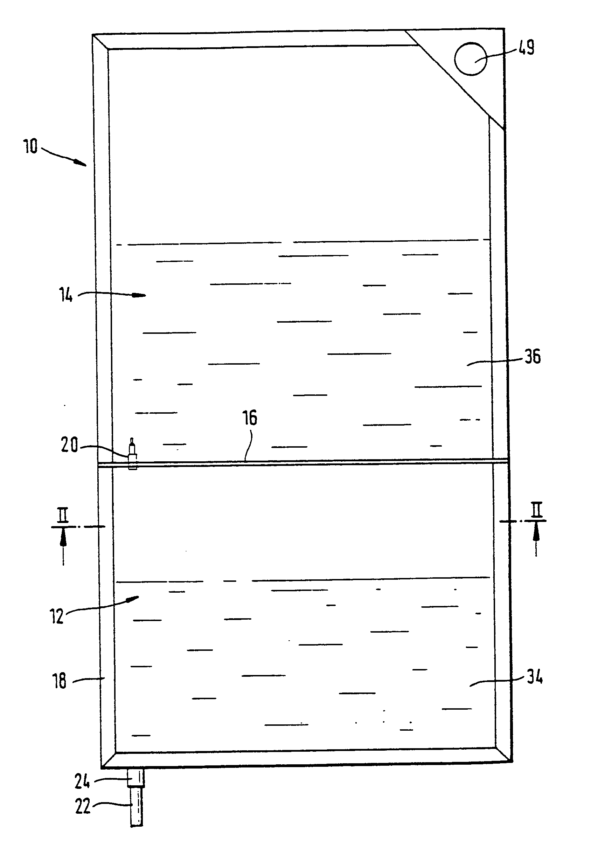

Fig. 1 einen Beutel in der Seitenansicht, Aus Fig. 1 und 2 ist ein Behälter 10 ersichtlich, der als Kunststoffbeutel ausgebildet ist. Dieser Behälter 10 weist zwei Kammern auf, nämlich eine erste Kammer 12 und eine zweite Kammer 14, die durch eine Septum 16 in Form einer Schweißnaht voneinander getrennt sind.

Der Beutel 10 weist weiterhin eine verschweißte Randzone 18 auf, mit der die beiden Kammern 12 und 14 gegenüber der Atmosphäre abgeschlossen sind. Diese Schweißnaht 18 ist im übrigen mit der Schweißnaht 16 verbunden, so daß auch zwischen den Kammern keine Strömungsverbindung mit Ausnahme des Strömungsverbindungsteils 20 besteht, das in die Schweißnaht 16 eingesetzt ist und von dieser umgeben ist.

Des weiteren ist die erste Kammer 12 mit einer Ablaßleitung 22 verbunden, die vorteilhafterweise won dem Schweißrand 18 umschweißt ist und mit der ersten Kammer eine Strömungsverbindung herstellen kann, sofern die vorteilhafterweise die Ablaßleitung 22 absperrende Absperreinrichtung 24 geöffnet wird. Diese besteht üblicherweise aus einem Kunststoffrohr mit einem angesetzten Abbrechteil, das beim Einsatz zerstört wird.

Das Strömungsverbindungsteil 20 besteht aus einem hohlzylindrischen Teil, das in eine weiteres hohlzylindrisches Teil mit geringerem Außendurchmesser übergeht und das mit einem Abbrechstück entlang der Schwächungslinie verschlossen ist.

Die erste Kammer 12 ist vorteilhafterweise mit einer bicarbonathaltigen, noch zu verdünnenden Flüssigkeit 34 gefüllt, während die zweite Kammer 14 mit einer Säurelösung 36 gefüllt ist. Beim Einsatz wird das Abbrechstück von dem Strömungsverbindungsteil 20 abgebrochen, so daß nunmehr die saure Lösung 36 durch den Strömungskanal 38 in dem Strömungsverbindungsteil 20 in die erste Kammere 12 strömen kann.

Nach der Vermischung der beiden Flüssigkeiten und der Herstellung der einzusetzenden Dialysierflüssigkeit bzw. Ersatzflüssigkeit für die Hämofiltration bzw. Infusionsflüssigkeit wird die Absperreinrichtung 24 geöffnet und somit die Ablaßleitung 22 freigegeben. Diese ist an ihrem anderen Ende mit einer üblichen, nicht gezeigten Anschlußeinrichtung, beispielsweise einem CAPD-Konnektorteil, einem Katheter, Infusionseinrichtung u.dgl., verbunden.

Schließlich weist der Behälter 10 an seinem oberen ende eine abgeschweißte Aufhängeeinrichtung 49 in Form einer Öse auf.

Wie bereits vorstehend erwähnt, werden die Lösungen durch nicht gezeigte Füllschlitze im Schweißrand 18 in die Kammern 12 und 14 eingefüllt, die anschließend zugeschweißt werden. Gegebenenfalls wird noch vor dem Zuschweißen zur bicarbonathaltigen Lösung in der ersten Kammer 12 eine bestimmte Menge gasförmiges $CO_2$ zugesetzt, beispielsweise um einen Innendruck von etwa 40—80 mm/Hg zu erzeugen, um das Zersetzungsgleichgewicht des Bicarbonats zu beeinflussen.

Des weiteren kann der Behälter 10 im Bereich der ersten Kammer 12 eine nichtgezeigte Zuspritzeinrichtung aufweisen, wie dies bei den derzeit eingesetzten CAPD-Beuteln üblicherweise der Fall ist.

Beispiel

Es wurden Behälter 10 hergestellt, die in der ersten Kammer 12 1500 ml bicarbonathaltige Lösung und in der zweiten Kammer 14 500 ml Säurelösung, insgesamt also 2 Liter Bicarbonatlösung enthielten. Diese gefüllten Zwei-Kammer-Beutel wurden in der üblichen Weise bei etwa 120°C sterilisiert und anschließend fü mehr als 6 Monate gelagert, wobei die unvermischten Lösungen stabil blieben und deren chemische Zusammensetzung sich nicht geändert hat. Untersuchungen auf Sterilität, Pyrogene und Partikel, die an den unvermischten und vermischten Lösungen durchgeführt worden sind, waren ebenfalls negativ. Weiterhin karamelisierte die der sauren Lösung zugesetzte Glucose nicht.

Schließlich war die vermischte Lösung noch 4 Tage nach dem Vermischen stabil.

Die vermischte Lösung hatte folgende Zusammensetzung in mval/l:

$Na^+$: 138

$K^+$: 1

$Ca^{2+}$: 4

$Mg^{2+}$: 1

$Cl^-$: 104

$HCO_3^-$: 35

Acetat: 5.

Des weiteren enthielt die Lösung 16,5 g/l Glucose, was einer Osmolarität von 369 mosm/l entspricht.

Es wurden 100 Beutel untersucht, deren vermischte Lösungen einen mittleren pH-Wert von etwa 7,2 und einen mittleren $CO_2$-Partialdruck $P_{CO_2}$ von etwa 85 mm/Hg aufwiesen.

Es wurden 4 × 2 Liter-Behälter bei CAPD-Patienten täglich verwendet, deren Behandlung mit dieser bicarbonathaltigen Lösung positiv verlief.

**Patentansprüche**

1. Behälter zur Bereitstellung einer bicarbonathaltigen Dialysier-, Substitutions- oder Infusionsflüssigkeit für die Peritonealdialyse, Hämofiltration bzw. Infusion, aufweisend einen ersten Behälterteil, der eine Säurelösung aufweist, und einen zweiten Behälterteil, der mit dem ersten Behälterteil über ein abgesperrtes, jedoch zu öffnendes Strömungsverbindungsteil verbunden ist und der mit einer bicarbonathaltigen Lösung gefüllt ist, wobei einer der Behälterteile wenigstens ein Auslaßrohr aufweist, das mit einem entfernbaren Verschluß versehen ist, dadurch gekennzeichnet, daß der erste Behälterteil und der zweite Behälterteil in einer wenigstens zwei Kammern (12, 14) aufweisenden Behälteranordnung (10) aus einem organischen Polymerisat vorgesehen sind.

2. Behälter nach Anspruch 1, dadurch gekennzeichnet, daß der Behälter (10) ein Zwei-Kammer-Beutel ist, dessen erste Kammer (12) von der zweiten Kammer (14) durch ein Septum (16) getrennt ist.

3. Behälter nach Anspruch 2, dadurch gekennzeichnet, daß das Septum als quer über den Beutel verlaufende Schweißnaht (16) ausgebildet ist, in der ein zu öffnendes Strömungsverbindungsteil (20) vorgesehen ist.

4. Behälter nach Anspruch 3, dadurch gekennzeichnet, daß die bicarbonathaltige Flüssigkeit (34) in der ersten Kammer (12), von der die Ablaßleitung (22) abgeht, und die Säureflüssigkeit (36) in der zweiten Kammer (14) der Behälteranordnung (10) vorliegen.

5. Behälter nach Anspruch 1, dadurch gekennzeichnet, daß als Innenfolie für den Zwei-Kammer-Beutel organische Polymere mit einer geringen Wasserdampfdurchlässigkeit und Kohlendioxiddurchlässigkeit eingesetzt werden.

6. Behälter nach Anspruch 5, dadurch gekennzeichnet, daß als Innenfolie Polyethylen oder PVC eingesetzt werden.

7. Behälter nach Anspruch 5, dadurch gekennzeichnet, daß die Innenfolie mit einer Außenfolie unter Bildung eines Laminats kaschiert ist.

8. Behälter nach Anspruch 7, dadurch gekennzeichnet, daß als Außenfolie Polyamide, PVC, Polyvinylidenchlorid, Polyethylenterephthalat oder Polyester eingesetzt werden.

9. Behälter nach Anspruch 8, dadurch gekennzeichnet, daß die Laminate eine Wasserdampfdurchlässigkeit nach Din 53122 von höchstens 1 und eine Kohlendioxiddurchlässigkeit von höchstens 20 cm³/m² × Tag × bar Druckdifferenz aufweisen und die Innenfolie eine Stärke von etwa 50—100 µm und die Außenfolie eine Stärke von etwa 20—100 µm aufweist.

10. Behälter nach Anspruch 3, dadurch gekennzeichnet, daß das Strömungsverbindungsteil (20) wenigstens ein hohlzylindrisches Teil aus einem Hartkunststoff aufweist, das über eine Schwächungslinie mit einem Abbrechstück verbunden ist.

11. Behälter nach Anspruch 1, dadurch gekennzeichnet, daß die Behälteranordnung (10) mit einer beutelförmigen Schutzhülle umgeben ist.

**Revendications**

1. Récipient pour la préparation d'un liquide de dialyse, de substitution ou de perfusion contenant du bicarbonate pour la dialyse péritonéale, l'hémofiltration ou la perfusion, comprenant une première partie de récipient qui contient une solution d'acide, et une deuxième partie de récipient, qui est raccordée à la première partie de récipient par un organe de raccordement d'écoulement fermé, mais pouvant être ouvert, et qui est remplie d'une solution contenant du bicarbonate, l'une des parties de récipient comprenant au moins un tube de sortie qui est muni d'un organe d'obturation amovible, caractérisé en ce que la première partie de récipient et la deuxième partie de récipient sont prévues dans un dispositif récipient (10) en polymère organique comprenant au moins deux chambres (12, 14).

2. Récipient suivant la revendication 1, caractérisé en ce que le récipient (10) est un poche à deux chambres, dont la première chambre (12) est séparée de la deuxième chambre (14) par un cloison (16).

3. Récipient suivant la revendication 2, caractérisé en ce que la cloison est formée par une ligne de soudure (16) établie transversalement sur la poche et dans laquelle est prévu un organe de raccordement d'écoulement (20) à ouvrir.

4. Récipient suivant la revendication 3, caractérisé en ce que le liquide contenant du bicarbonate (34) est contenu dans la première chambre (12), d'où part la conduite de sortie (22), et la solution d'acide (36) est contenue dans la deuxième chambre (14) du dispositif récipient (10).

5. Récipient suivant la revendication 1, caractérisé en ce que des polymères organiques ayant une faible perméabilité à la vapeur d'eau et une faible perméabilité au dioxyde de carbone sont utilisés comme feuille intérieure pour la poche à deux chambres.

6. Récipient suivant la revendication 5, caractérisé en ce que le polyéthylène ou le PVC sont utilisés comme feuille intérieure.

7. Récipient suivant la revendication 5, caractérisé en ce que la fueille intérieure est doublée d'une feuille extérieure avec formation d'un stratifié.

8. Récipient suivant la revendication 7, caractérisé en ce que des polyamides, le PVC, le poly(chlorure de vinylidène), le poly(téréphtalate d'éthylène) ou des polyesters sont utilisés comme feuille extérieure.

9. Récipient suivant la revendication 8, caractérisé en ce que les stratifiés ont une perméabilité à la vapeur d'eau suivant DIN 53122 de 1 au maximum et une perméabilité au dioxyde de carbone de 20 cm³ par m² par jour et par bar de différence de pression au maximum, la feuille intérieure a une épaisseur d'environ 50 à 100 μm et la feuille extérieure, une épaisseur d'environ 20 à 100 μm.

10. Récipient suivant la revendication 3, caractérisé en ce que l'organe de raccordement d'écoulement (20) comprend au moins un corps cylindrique creux fait d'une matière plastique dure, qui est raccordé par un ligne d'affaiblissement à une pièce à rompe.

11. Récipient suivant la revendication 1, caractérisé en ce que le dispositif récipient (10) est entouré d'un voile de protection en forme de poche.

**Claims**

1. A container for preparing a bicarbonate-containing solution for dialysis, substitution or infusion for peritoneal dialysis, hemofiltration or respectively infusion, comprising a first chamber with an acid solution, and a second chamber which is connected with said first chamber via a closed flow passage part, which may, however, be opened, and which is filled with a bicarbonate-containing solution, with one of the chambers comprising at least one discharge tube fitted with a removable closure, characterized in that said first chamber and said second chamber are provided within a container assembly (10) made from an organic polymer having a least two chambers (12, 14).

2. Container according to claim 1, characterized in that said container (10) is a two-chamber-bag whose first chamber (12) is separated from the second chamber (14) by a septum (16).

3. Container according to claim 2, characterized in that the septum (16) as embodied as weld seam extending transverse over the bag and in which a flow passage part (20) is provided.

4. Container according to claim 2, characterized in that the bicarbonate-containing fluid (34) is present in the first chamber (12) from which the discharge line (22) departs, and the acid solution (36) is present in the second chamber (14) of the container assembly.

5. Container according to claim 1, characterized in that as inner foil for the two-chamber-bag organic polymers having a low water-vapor permeability and carbon dioxide permeability are used.

6. Container according to claim 5, characterized in that as inner foil polyethylene or PVC are used.

7. Container according to claim 5, characterized in that the inner foil is covered with an outer foil to form a laminate.

8. Container according to claim 7, characterized in that as outer foil polyamide, PVC, polyvinalidene chloride, polyethylene terephthalate or polyester may be used.

9. Container according to claim 8, characterized in that the laminates have a water-vapor permeability according to DIN 53122 of at least 1 and a permeability for carbon dioxide of at least 20 $cm^3/m^2 \times day \times bar$ pressure difference and that the inner foil has a thickness of approximately 50—100 μm and the outer foil a thickness of approximately 20—100 μm.

10. Container according to claim 3, characterized in that the flow passage part (20) comprises at least one hollow cylindrical portion made from hard resin which is connected with a break-off part by way of a line of weakness.

11. Container according to claim 1, characterized in that the container assembly (10) is surrounded by a bag-shaped protective layer.